# EUROPEAN PATENT SPECIFICATION

(11) **EP 2 014 227 B1**
(45) Date of publication and mention of the grant of the patent: **24.11.2010**
(21) Application number: 07740816.9
(22) Date of filing: 02.04.2007
(51) Int. Cl.: A61B 5/0245, G01L 1/14, G01D 5/24, G01R 27/26

(54) **PULSE WAVE MEASURING DEVICE**
PULSWELLENMESSER
DISPOSITIF DE MESURE DES ONDES DE PRESSION

(30) Priority: 26.04.2006 JP 2006122206
(43) Date of publication of application: 14.01.2009
(73) Proprietor: Omron Healthcare Co., Ltd., Kyoto-shi, Kyoto 615-0084 (JP)
(72) Inventor: TANABE, Kazuhisa c/o OMRON HEALTHCARE CO., LTD., Kyoto-shi, Kyoto 615-0084 (JP)
(74) Representative: Wilhelms · Kilian & Partner Patentanwälte
(86) International application number: PCT/JP2007/057380
(87) International publication number: WO 2007/125728

(56) References cited:
- DE-A1- 4 042 335
- JP-A- 08 327 677
- JP-A- 09 072 757
- JP-A- 2006 020 823
- PARK AND K D WISE Y E: "AN MOS SWITCHED-CAPACITOR READOUT AMPLIFIER FOR CAPACITIVE PRESSURE SENSORS" PROCEEDINGS IEEE CUSTOM CIRCUITS CONFERENCE,, 1 May 1983 (1983-05-01), pages 380-384, XP009117916

## Description

### TECHNICAL FIELD

The present invention relates to a pulse-wave measurement apparatus and, more particularly, relates to a pulse-wave measurement apparatus for measuring pressure waveforms in an artery using a capacitance device.

### BACKGROUND ART

As a pressure-pulse-wave measurement method for easily obtaining pressure waveforms in an artery in a bloodless manner, there has been known a tonometry method described in G.L.Pressman, P.M.Newgard, "A Transducer for the Continuous External Measurement of Arterial Blood Pressure", IEEE TRANSACTIONS ON BIO-MEDICAL ELECTRONICS, 1963, pp. 74-81 (Non-Patent Document 1). In the tonometry method, a solid flat plate is pressed against a biologic surface to press the biologic surface by the solid flat plate to such an extent that a flat portion is formed in the artery. Further, a pressure equilibrium state where the influence of the tension occurring at the surface of the artery is eliminated is maintained, and only the pressure change in the artery is stably measured with higher accuracy.

In recent years, attempts have been made to determine the state within a biologic body, by calculating a characteristic amount from pressure waveforms in an artery which have been measured according to the tonometry method. As one of these attempts, studies have been earnestly conducted for Al (Augmentation Index) values as indexes for determining the degrees of sclerosis of arteries.

As a condition required for measuring pressure waveforms in an artery using the tonometry method, there is a need for placing a sensor device just above a flat portion formed in the artery, in addition to pressing the biologic surface to such an extent that such a flat portion is formed in the artery. Further, in order to measure pressure waveforms in the artery with higher accuracy, there is a need for structuring the sensor device to have a width smaller than the width of the flat portion formed in the artery, which requires the sensor device to have a size sufficiently smaller than the artery diameter. In consideration of the aforementioned circumstances, it is significantly hard to position and place a single sensor device just above the flat portion formed in the artery. Accordingly, it is practical to place a pressure sensor constituted by plural micro-fabricated sensor devices substantially orthogonally to the direction of the extension of the artery, for measuring pressure pulse waves.

In general, as sensing methods for measuring pressures, there are known sensing methods utilizing a distortion resistance device and sensing methods utilizing a capacitance device. The sensing methods utilizing a capacitance device offer the advantage of fabrication with lower costs without using semiconductor processes which require higher fabrication costs, since the sensor devices have a simple structure, in comparison with a distortion resistance device.

For example, there has disclosed an electric-charge-to-voltage conversion type sensor device constituted by an amplifier, a capacitor, a switch and the like, in Y.E.Park and K.D.Wise, "AN MOS SWITCHED-CAPACITOR READOUT AMPLIFIER FOR CAPACITIVE PRESSURE SENSORS", Proc.IEEE Custom Circuit Conf., May 1983, pp. 380-384 (Non-Patent Document 2).

Here, in the case of using a pressure sensor having plural sensor devices placed therein as described above, there is a need for a multiplexer for selecting the outputs of the plural sensor devices. It is necessary to fabricate the multiplexer, through MOS (Metal Oxide Semiconductor) processing, in general. Since MOS processing is employed for the sensor device described in Non-Patent Document 2, it is possible to standardize fabrication processing even in cases where a multiplexer is required, thereby enabling reduction of the size of the sensor device.

The sensor device described in Non-Patent Document 2 can have a reduced size, as described above, and also consumes less electric power, since it employs MOS processing. Accordingly, the sensor device described in Non-Patent Document 2 has been employed in MEMS (Micro Electro Mechanical System) pressure sensors and MEMS acceleration sensors.
[Non-Patent Document 1] G.L.Pressman, P.M.Newgard, "A Transducer for the Continuous External Measurement of Arterial Blood Pressure", IEEE TRANSACTIONS ON BIO-MEDICAL ELECTRONICS, 1963,
[Non-Patent Document 2] Y.E.Park and K.D.Wise, "AN MOS SWITCHED-CAPACITOR READOUT AMPLIFIER FOR CAPACITIVE PRESSURE SENSORS", Proc.IEEE Custom Circuit Conf., May 1983, pp. 380-384
Non-Patent Document 2 and DE 40 42 335 A1 each form the basis for the preamble of claim 1.

### DISCLOSURE OF THE INVENTION

### PROBLEMS TO BE SOLVED BY THE INVENTION

On the other hand, in the sensor device described in Non-Patent Document 2 and in DE 40 42 335 A1, the output and the input of the amplifier are connected to each other through the capacitor and the switch placed in parallel to form a feedback circuit. By switching this switch, the amplifier is caused to output, as a sensor output, a voltage corresponding to the electric charge accumulated in the capacitance device, namely a voltage corresponding to the capacitance of the capacitance device, In this case, a MOS analog switch, for example, is used as the switch constituting the feedback circuit.

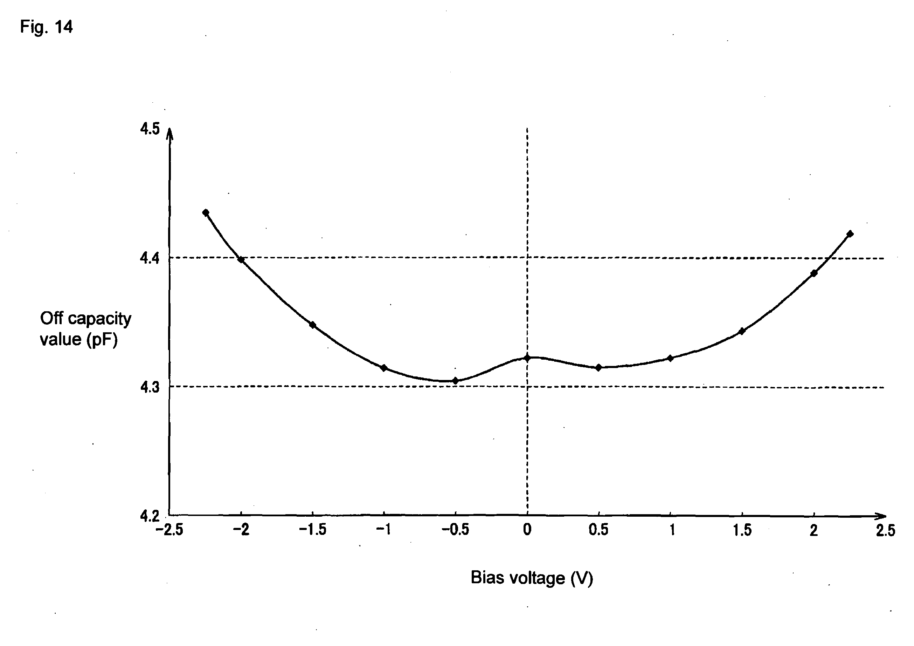

Fig. 14 is a view illustrating an example of the bias-voltage dependence of the off capacity of the MOS analog switch.

Referring to Fig. 14, the MOS analog switch has an off capacity of several pF even at an OFF state, and the off capacity is changed depending on the bias voltage applied between the opposite ends of the switch.

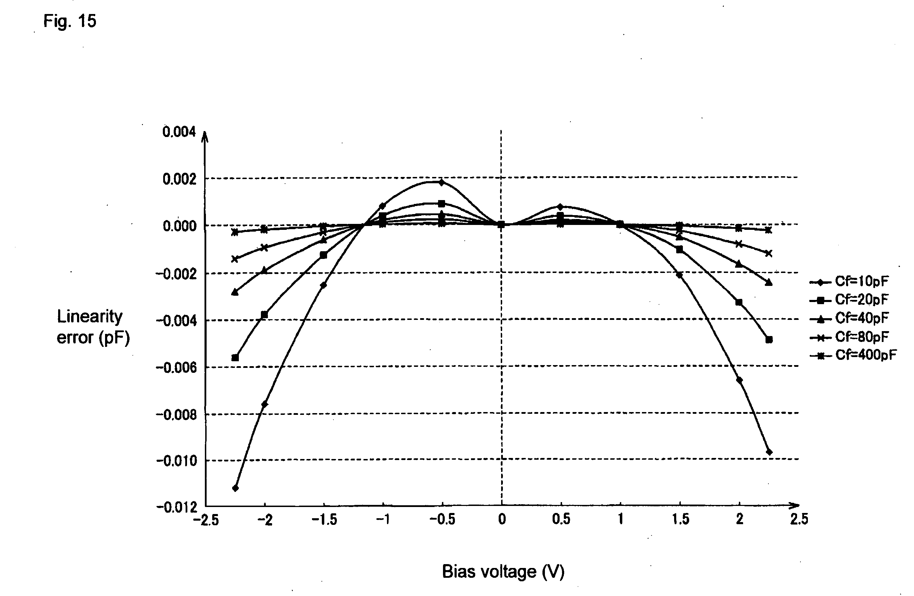

Fig. 15 is a view illustrating linearity errors in the case of using a MOS analog switch having the bias-voltage dependence illustrated in Fig. 14, in an electric-charge-to-voltage conversion type sensor device.

Referring to Fig. 15, in an electric-charge-to-voltage conversion type sensor device, such as the sensor device described in Non-Patent Document 2 and in DE 40 42 335 A1, the output voltage from the amplifier, namely the output characteristic of the sensor device, is nonlinear, due to the bias-voltage dependence of the off capacity of the switch constituting the feedback circuit, such as a MOS analog switch. Particularly, a pulse-wave measurement apparatus for measuring pressure waveforms in an artery employs a fine sensor device as described above and therefore, in order to ensure excellent sensing sensitivity, it is necessary to cause the capacitor constituting the feedback circuit to have a smaller capacity, thereby increasing the error in the sensor output due to the off capacity of the MOS analog switch.

Therefore, it is an object of the present invention to provide a pulse-wave measurement apparatus capable of preventing the nonlinearity of the output characteristic and reducing the error of pulse-wave detection.

### MEANS FOR SOLVING THE PROBLEMS

A pulse-wave measurement apparatus according to an aspect of the present invention is a pulse-wave measurement apparatus for measuring pressure pulse waves in an artery by being pressed against a biologic surface, and includes a pressure detecting capacitor which changes its capacitance according to the pressure within the artery, an operational amplifier which is connected at its inverting input terminal to one end of the pressure detecting capacitor and also is connected at its non-inverting input terminal to a reference voltage, a charge transfer capacitor which is connected at its one end to the inverting input terminal of the operational amplifier and, also, is connected at the other end to the output of the operational amplifier, a first switch which is connected at its one end to the inverting input terminal of the operational amplifier, and a voltage setting portion which is connected at its one end to the other end of the first switch and also is connected at the other end to the output of the operational amplifier, wherein the voltage setting portion connects the other end of the first switch to the output of the operational amplifier or disconnects the other end of the first switch from the output of the operational amplifier and applies a predetermined voltage to the other end of the first switch.

Preferably, the voltage setting portion includes a second switch which is connected at its one end to the other end of the first switch and also is connected at the other end to the output of the operational amplifier, and a third switch which is connected at its one end to the other end of the first switch and also is connected at the other end to the predetermined voltage.

Preferably, the voltage setting portion includes a second switch which is connected at its one end to the other end of the first switch and also is connected at the other end to the output of the operational amplifier, and a voltage setting capacitor which is connected at its one end to the other end of the first switch and also is connected at the other end to the predetermined voltage.

Preferably, the pulse-wave measurement apparatus further includes a charging portion for applying a charging voltage to the other end of the pressure detecting capacitor and a control portion, wherein the control portion applies the charging voltage to the other end of the pressure detecting capacitor, turns on the first switch and connects the other end of the first switch to the output of the operational amplifier, then disconnects the other end of the first switch from the output of the operational amplifier, then applies the predetermined voltage to the other end of the first switch and also stops the application of the charging voltage, by controlling the charging portion, the first switch and the voltage setting portion.

Preferably, the pulse-wave measurement apparatus further includes a fourth switch which is connected at its one end to one end of the pressure detecting capacitor and also connected at the other end to the inverting input terminal of the operational amplifier.

More preferably, the pulse-wave measurement apparatus further includes a charging portion for applying a charging voltage to the other end of the pressure detecting capacitor and a control portion, wherein the control portion applies the charging voltage to the other end of the pressure detecting capacitor, turns on the first switch, turns on the fourth switch, connects the other end of the first switch to the output of the operational amplifier, then turns off the fourth switch, then disconnects the other end of the first switch from the output of the operational amplifier, then applies the predetermined voltage to the other end of the first switch, also stops the application of the charging voltage and turns on the fourth switch, by controlling the charging portion, the first switch and the fourth switch and the voltage setting portion.

Preferably, the predetermined voltage is a reference voltage.

### EFFECT OF THE INVENTION

According to the present invention, it is possible to prevent the nonlinearity of the output characteristic, thereby reducing the error of the pulse-wave detection.

### BRIEF DESCRIPTION OF THE DRAWINGS

Fig. 1 is an external view of a pulse-wave measurement apparatus according to a first embodiment of the present invention.
Fig. 2 is a schematic cross-sectional view of the carpus and the pulse-wave measurement apparatus at the measurement state illustrated in Fig. 1.
Fig. 3 is a view illustrating the structures of a sensor array 19, a multiplexer 20 and a C-V conversion portion 21 in the pulse-wave measurement apparatus according to the first embodiment of the present invention.
Fig. 4 is an external perspective view of the sensor array 19.
Fig. 5 is a functional block diagram of the pulse-wave measurement apparatus according to the first embodiment of the present invention.
Fig. 6 is a flow chart defining operations and procedures when the pulse-wave measurement apparatus according to the first embodiment of the present invention measures pulse waves.
Fig. 7 is a functional block diagram illustrating the structures of the C-V conversion portion 21 and a capacitor CX in the pulse-wave measurement apparatus according to the first embodiment of the present invention.
Fig. 8 is a circuit diagram illustrating the structures of the C-V conversion portion 21 and a capacitor CX in the pulse-wave measurement apparatus according to the first embodiment of the present invention.
Fig. 9 is a timing chart illustrating the operations of the C-V conversion portion 21 when the pulse-wave measurement apparatus according to the first embodiment of the present invention performs pulse wave measurement.
Fig. 10 is a flow chart defining the operations and procedures of the C-V conversion portion 21 when the pulse-wave measurement apparatus according to the first embodiment of the present invention performs pulse wave measurement.
Fig. 11 is a circuit diagram illustrating the structures of the C-V conversion portion 21 and a capacitor CX in the pulse-wave measurement apparatus according to a second embodiment of the present invention.
Fig. 12 is a timing chart illustrating the operations of the C-V conversion portion 21 when the pulse-wave measurement apparatus according to the second embodiment of the present invention performs pulse wave measurement.
Fig. 13 is a flow chart defining the operations and procedures of the C-V conversion portion 21 when the pulse-wave measurement apparatus according to the second embodiment of the present invention performs pulse wave measurements.
Fig. 14 is a view illustrating an example of the bias-voltage dependence of the off capacity of a MOS analog switch.
Fig. 15 is a view illustrating linearity errors in the case of using a MOS analog switch having the bias-voltage dependence illustrated in Fig. 14, in an electric-charge-to-voltage conversion type sensor device.

### EXPLANATION OF SYMBOLS

1: sensor unit, 3: display unit, 11: CPU (control portion), 12: ROM, 13: RAM, 14: driving circuit, 15: pressure pump, 16: negative-pressure pump, 17: change-over valve, 18: pressing cuff, 19: sensor array, 20: multiplexer, 21: C-V conversion portion, 22: low-pass filter, 23: A/D conversion portion, 24: operation portion, 25: display portion, 28 and 28A: sensor element, 30: spacer member, 31: lower electrodes, 32: upper electrode, 51: charging portion, 52: voltage conversion portion, 53: voltage holding portion, 54: voltage setting portion, 100: pulse-wave measurement apparatus, 110: placement table, 122: casing, 130: fastening belt, 200: arm, 210: artery, 220: radius, CX: capacitor (pressure detecting capacitor), CC and. CH1: capacitor, CF: charge transfer capacitor, CS: capacitor (voltage setting capacitor), SW1 to SW4: switch (first to fourth switches), SW5, SW51 to SW54; switch, G1 and G2: operational amplifier, V1 and V2: power supply.

### PREFERRED EMBODIMENTS FOR CARRYING OUT THE INVENTION

Hereinafter, embodiments of the present invention will be described with reference to the drawings. Herein, same reference numerals are denoted for the same or corresponding components throughout the drawings, and the description thereof will not be repeated.

### <First Embodiment>

### [The Structure and Basic Operations of Pulse-Wave Measurement Apparatus]

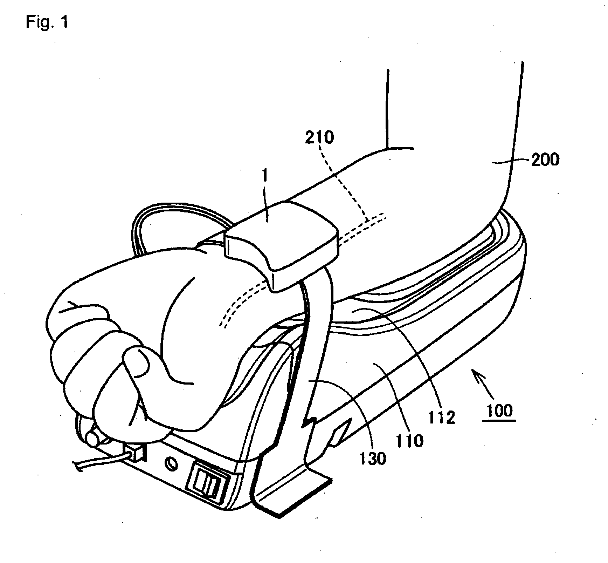

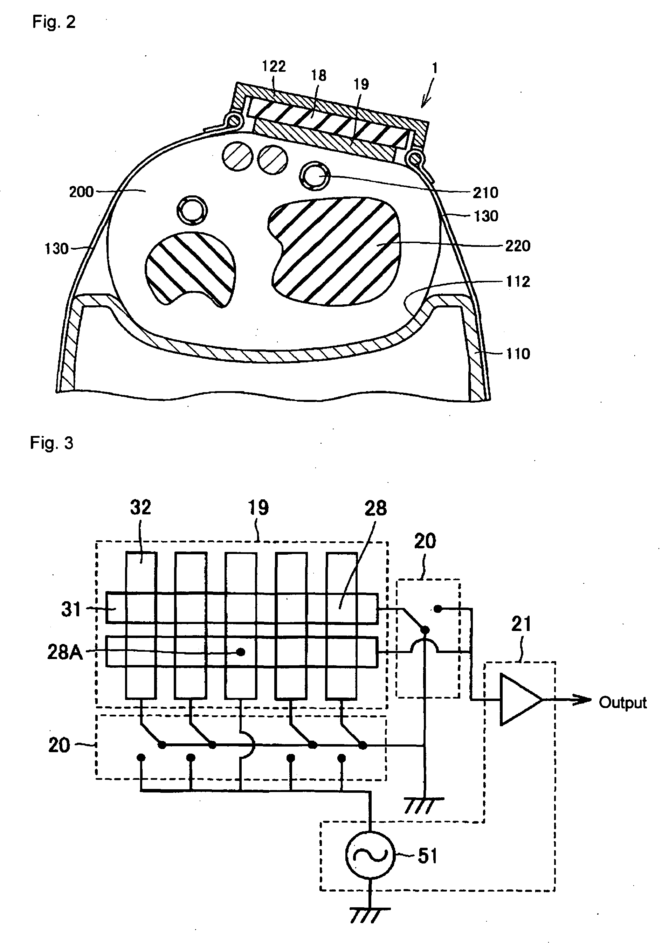

Fig. 1 is an external view of a pulse-wave measurement apparatus according to the first embodiment of the present invention. Further, Fig. 1 illustrates a measurement state where a sensor array is pressed against a carpus. Fig. 2 is a schematic cross-sectional view of the carpus and the pulse-wave measurement apparatus at the measurement state illustrated in Fig. 1.

Referring to Figs. 1 and 2, the pulse-wave measurement apparatus 100 is for measuring pressure waveforms in an artery in a carpus of a to-be-tested person. The pulse-wave measurement apparatus 100 includes a placement table 110, a sensor unit 1 and a fastening belt 130. The sensor unit 1 includes a casing 122, a pressing cuff 18 and a sensor array 19.

The placement table 110 includes a placement portion 112 for placing the carpus and the forearm of one arm 200 of the to-be-tested person. The fastening belt 130 secures the carpus portion of the arm 200 placed on the placement table 110. The sensor unit 1 is mounted to the fastening belt 130 and incorporates the sensor array 19.

Referring to Fig. 1, at a state where the carpus is secured to the placement table 110, the artery 210 is positioned in the direction parallel to the direction in which the arm 200 extends. Referring to Fig. 2, the pressing cuff 18 incorporated in the casing 122 in the sensor unit 1 is expanded, which descends the sensor array 19, thereby pressing the sensor surface of the sensor array 19 against the surface of the carpus. The inner pressure of the pressing cuff 18 is adjusted by a pressure pump 15 and a negative-pressure pump 16 which will be described later. The sensor array 19 is placed, such that lower electrodes 31 provided on the sensor surface, which will be described later, are extended substantially orthogonally to the direction in which the artery 210 extends.

During the pressing, the artery 210 is vertically sandwiched between the radius 220 and the sensor surface of the sensor array 19, thereby forming a flat portion in the artery 210. Further, at least one sensor element 28 is positioned just above the flat portion formed in the artery 210.

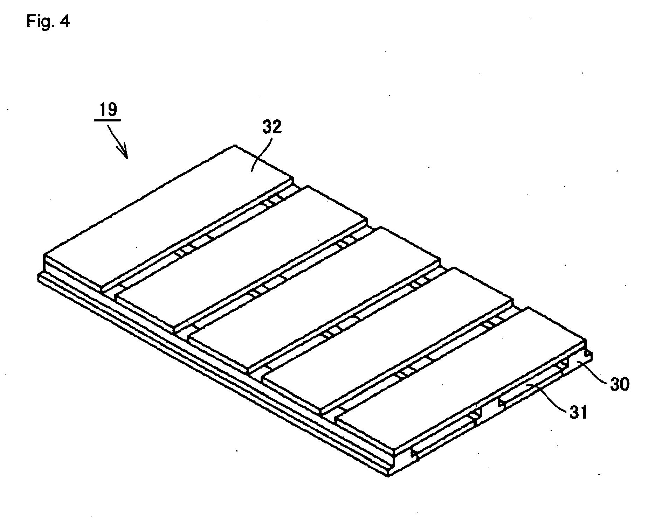

Fig. 3 is a view illustrating the structures of the sensor array 19, a multiplexer 20 and a C-V conversion portion 21 in the pulse-wave measurement apparatus according to the first embodiment of the present invention. Fig. 4 is an external perspective view of the sensor array 19.

Referring to Fig. 3, the sensor array 19 is used, in combination with the multiplexer 20 and the C-V conversion portion 21. The C-V conversion portion 21 includes a charging portion 51.

Referring to Fig. 4, the sensor array 19 includes lower electrodes 31, upper electrodes 32 and spacer members 30. The lower electrodes 31 are constituted by plural band-shaped cupper-sheet electrodes extending substantially straightly which are provided in the form of rows, such that they are parallel to one another. The upper electrodes 32 are constituted by plural band-shaped cupper-sheet electrodes extending substantially straightly which are provided in the form of columns, such that they are parallel to one another, in the direction orthogonal to the lower electrodes 31. Between the lower electrodes 31 and the upper electrodes 32, there are placed the spacer members 30 made of a silicon rubber.

At the intersections of the lower electrodes 31 and the upper electrodes 32 which are placed in a matrix shape, the lower electrodes 31 and the upper electrodes 32 are placed, such that they are faced to each other and are spaced apart from each other by a predetermined distance by the spacer members 30. Accordingly, sensor elements 28 are formed at the intersections of the lower electrodes 31 and the upper electrodes 32. That is, the sensor array 19 includes the plural sensor elements 28 placed in the matrix shape.

The sensor elements 28 change their capacitances, when they are distorted in such a direction that the upper electrodes 32 and the lower electrodes 31 come close to each other due to pressures applied to the upper electrodes 32 and the lower electrodes 31.

Referring again to Fig. 3, the C-V conversion portion 21 is connected through the multiplexer 20 to one of the lower electrodes 31 and one of the upper electrodes 32. The multiplexer 20 selects a certain lower electrodes 31 and a certain upper electrode 32. With this structure, the capacitance of any one of the plural sensor elements 28 placed in the matrix shape can be obtained as an output voltage of the C-V conversion portion 21. For example, when the multiplexer 20 selects the lower electrode 31 in the second row from the top and the upper electrode 32 in the third column from the left, the sensor element 28A is connected to the C-V conversion portion 21. Accordingly, it is possible to determine the pressure waveform at an arbitrary position in the sensor array 19. Further, while, in Fig. 3, the upper electrodes 32 are connected to the charging portion 51 through the multiplexer 20, it is possible to employ a structure in which the lower portions 31 are connected to the charging portion 51 through the multiplexer 20 by reversing the connection relationship between the lower electrodes 31 and the upper electrodes 32.

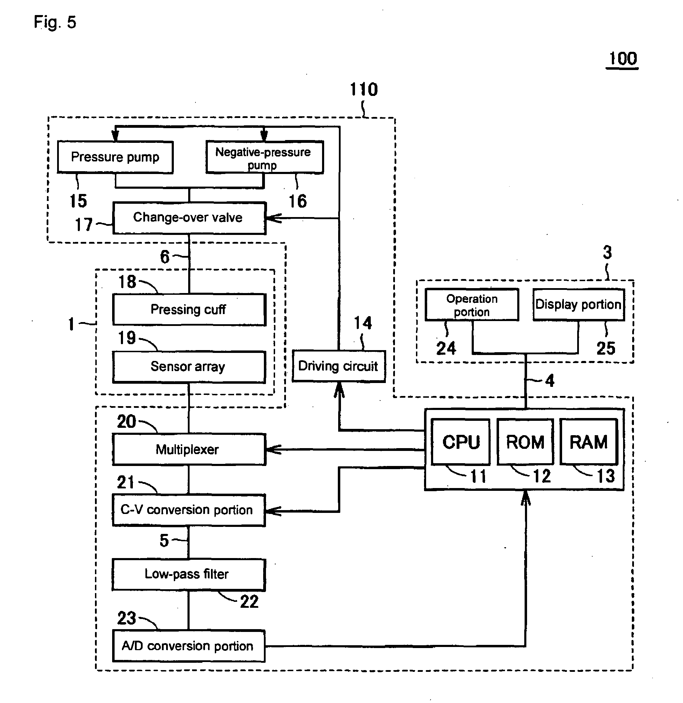

Fig. 5 is a functional block diagram of the pulse-wave measurement apparatus according to the first embodiment of the present invention.
Referring to Fig. 5, the pulse-wave measurement apparatus 100 includes the sensor unit 1, a display unit 3 and the placement table 110. The display unit 3 includes an operation portion 24 and a display portion 25. The sensor unit 1 includes the pressing cuff 18 and the sensor array 19. The placement table 110 includes a ROM (Read Only Memory) 12, a RAM (Random Access Memory) 13, a CPU (Central Professing Unit) (control portion) 11, a driving circuit 14, the pressure pump 15 and the negative-pressure pump 16, a change-over valve 17, the multiplexer 20, the C-V conversion portion 21, a low-pass filter 22, and an A/D conversion portion 23.

The operation portion 24 detects operations performed from the outside and outputs the results of detections, as operation signals, to the CPU 11. A user inputs various types of information on the pulse wave measurements to the pulse-wave measurement apparatus 100, by operating the operation portion 24.

The display portion 25 includes LEDs (Light Emitting Diodes) and an LCD (Liquid Crystal Display) for outputting various types of information, such as the results of detections of artery positions and the results of pulse-wave measurements to the outside.

The ROM 12 and the RAM 13 store, for example, data and programs for controlling the pulse-wave measurement apparatus 100.

The driving circuit 14 drives the pressure pump 15, the negative-pressure pump 16 and the change-over valve 17, based on control signals from the CPU 11.

The CPU 11 reads programs from the ROM 12 by accessing it, decompresses the read programs in the RAM 13 and executes the programs for performing control of the respective blocks in the pulse-wave measurement apparatus 100 and for performing calculation processing. Further, the CPU 11 performs processing for controlling the respective blocks in the pulse-wave measurement apparatus 100, based on user operation signals received from the operation portion 24. That is, the CPU 11 outputs control signals to the respective blocks, based on operation signals received from the operation portion 24. Further, the CPU 11 displays the results of pulse-wave measurements, on the display portion 25.

The pressure pump 15 is a pump for increasing the inner pressure of the pressing cuff 18, while the negative-pressure pump 16 is a pump for decreasing the inner pressure of the pressing cuff 18. The change-over valve 17 selectively connects one of the pressure pump 15 and the negative pressure pump 16 to an air pipe 6.

The pressing cuff 18 includes an air bag which is adjusted in pressure for pressing the sensor array 19 against the carpus.

The sensor array 19 is pressed against a to-be-measured portion such as a carpus of the to-be-tested person, through the pressure of the pressing cuff 18. The sensor array 19 detects pulse waves of the to-be-tested person, namely pressure waveforms in an artery, through the radial artery, when it is pressed.

The multiplexer 20 selects any one of the plural sensor elements 28 in the sensor array 19, based on control signals received from the CPU 11. The C-V conversion portion 21 converts the value of the capacitance of the sensor element 28 selected by the multiplexer 20 into a voltage, namely it outputs, as voltage signals (hereinafter, also referred to as pressure signals), pressure oscillation waves indicative of pressure waveforms in the artery which are transferred from the artery to the biologic surface.

The low-pass filter 22 attenuates predetermined frequency components, out of the pressure signals received from the C-V conversion portion 21.

The A/D conversion portion 23 converts the pressure signals which are the analog signals passed through the low-pass filter 22 into digital signals and outputs them to the CPU 11.

Herein, the placement table 110 can be structured to include the display unit 3. Further, although the placement table 110 is structured to include the CPU 11, the ROM 12 and the RAM 13, the display unit 3 can be structured to include them. Further, the CPU 11 can be structured to perform various types of control, by being connected to a PC (Personal Computer).

### [Operations of the Pulse-Wave Measurement Apparatus]

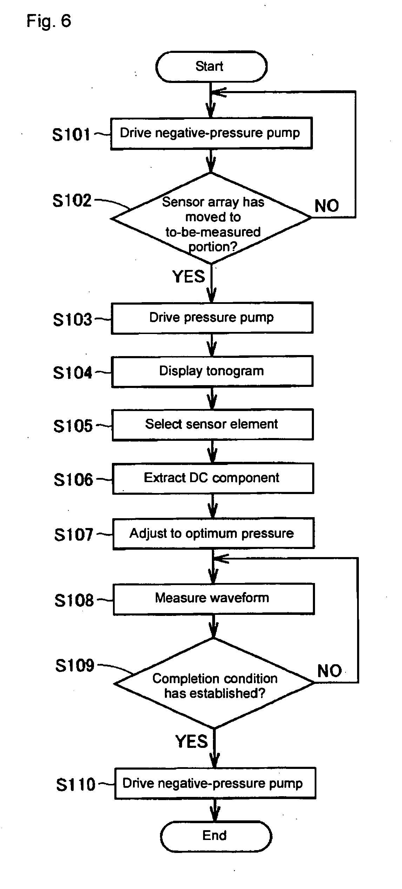

Fig. 6 is a flow chart defining the operations and procedures when the pulse-wave measurement apparatus according to the first embodiment of the present invention measures pulse waves. The CPU 11 reads programs from the ROM 22 by accessing it, decompresses the read programs in the RAM 23 and executes them to realize the processing illustrated in the flow chart of Fig. 6.

Referring to Fig. 6, at first, if the pulse-wave measurement apparatus 100 is powered up, the CPU 11 commands the driving circuit 14 to drive the negative-pressure pump 16. The driving circuit 14 changes over the change-over valve 17 to the negative-pressure pump 16 and drives the negative-pressure pump 16, based on the command from the CPU 11 (S101). The negative-pressure pump 16 being driven decreases the inner pressure of the pressing cuff 18 through the change-over valve 17 such that it is sufficiently lower than the atmospheric pressure. With this structure, it is possible to prevent the occurrence of false operations and failures due to accidental protrusion of the sensor array 19.

If the CPU 11 detects that the sensor array 19 has moved to a to-be-measured portion (S102), it starts pulse-wave measurement. In this case, the sensor unit 1 includes a micro switch for detecting the movement of the sensor array 19, which is not illustrated, and the CPU 11 determines the position of the sensor array 19 based on detection signals from the micro switch, Also, the CPU 11 can be structured such that it starts pulse-wave measurement, on detecting that a measurement-starting switch (not illustrated) included in the operation portion 24 has been pushed.

If the sensor array 19 has moved to the to-be-measured position (YES in S102), the CPU 11 commands the driving circuit 14 to drive the pressure pump 15. The driving circuit 14 changes over the change-over valve 17 to the pressure pump 15 and drives the pressure pump 15, based on the command from the CPU 11 (S103). The pressure pump 15 being driven increases the inner pressure of the pressing cuff 18 through the change-over valve 17 to press the sensor array 19 against the surface of the to-be-measured portion of the to-be-tested person.

When the sensor array 19 is pressed against the to-be-measured portion, the multiplexer 20 changes over the sensor element 28 connected to the C-V conversion portion 21, in a time-division manner, under control of the CPU 11. The C-V conversion portion 21 converts the value of the capacitance of the sensor element 28 selected by the multiplexer 20 into a voltage. The low-pass filter 22 attenuates predetermined frequency components, out of pressure signals received from the C-V conversion portion 21. The A/D conversion portion 23 converts the pressure signals passed through the low-pass filter 22 into digital information and outputs it to the CPU 11.

The CPU 11 creates a tonogram indicative of the relationship between the positions of the sensor elements 28 and the pressure signals, based on the digital information received from the A/D conversion portion 23 and displays it on the display portion 25 (S104).

The CPU 11 detects and selects the sensor element 28 existing above the artery, based on the created tonogram (S105). Further, the processing for detecting the sensor element 28 can be performed using the techniques described in JP-A No. 2004-222847 which has been already filed by the present applicant and published.

Further, the CPU 11 extracts the direct current (DC) component of the pressure signal outputted from the C-V conversion portion 21, based on the digital information received from the A/D conversion portion 23 (S106). The DC component of the pressure signal is expressed by the average value of the pressure signals over a predetermined interval; a pressure signal created by eliminating, from the pressure signal, components having frequencies lower than a predetermined frequency, namely pulse wave components, a pressure signal level at a pulse-wave rising point, namely just before pulse wave components are mixed therein.

More specifically, the DC component can be extracted by dividing the change of the outputted pressure signal into windows (sections) at predetermined intervals and then calculating the averages over the respective windows. Also, similarly, the DC component can be extracted by, for example, calculating the intermediate value between the maximum value and the minimum value in each window. Further, the aforementioned predetermined interval can be an interval which has been preliminarily set in the pulse-wave measurement apparatus 100 regardless of the pulse wave of the to-be-tested person and is preferably about 1.5 seconds which is equal to or more than intervals of general pulse waves.

Next, the CPU 11 performs optimum pressure adjustment; namely adjusts the inner pressure of the pressing cuff 18 such that the DC component of the pressure signal is stabilized, by controlling the driving circuit 14 (S107).

Next, the CPU 11 acquires waveform data, based on the currently-selected pressure signal from the C-V conversion portion 21 which is indicated by the digital information received from the A/D conversion portion 23, and determines the pulse wave based on the acquired waveform data (S108).

Further, if a pulse-wave measurement completion condition is established (YES in S109), the CPU 11 drives the negative-pressure pump 16 to release the pressing of the sensor array 19 against the to-be-measured portion, by controlling the driving circuit 14 (S110). In this case, the pulse-wave measurement completion condition can be the elapse of a preset predetermined time interval (for example, 30 seconds) or a command for the completion of measurement or a command for interruption of measurement from the user.

On the other hand, if the predetermined condition has not established (NO in S109), the CPU 11 repeatedly performs waveform-data transfer processing to continue the pulse-wave measurement (S108).

### [The Structure and Basic Operations of the C-V Conversion Portion and the Sensor Elements]

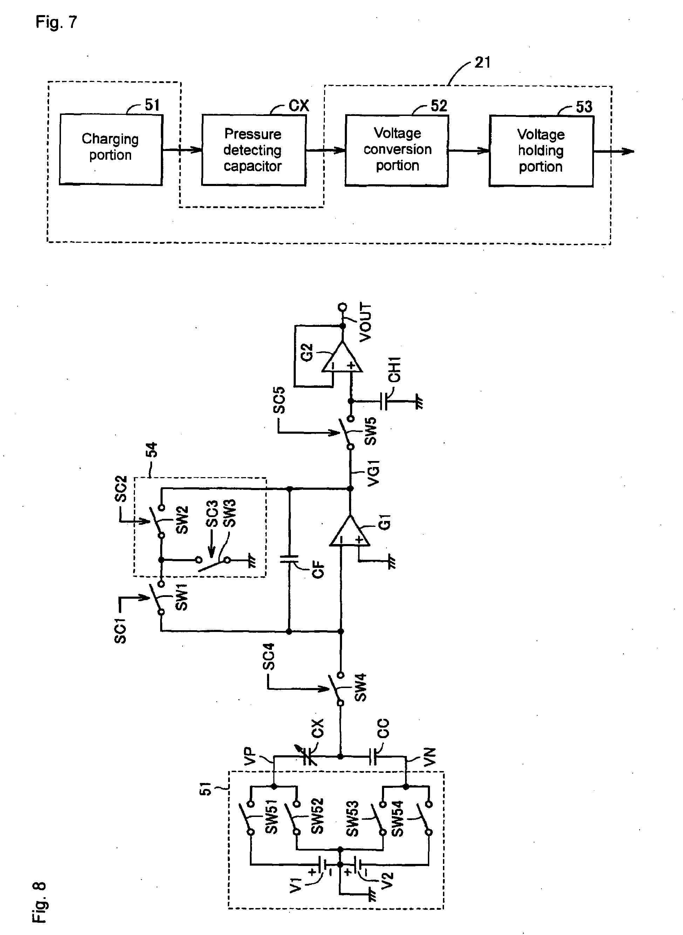

Fig. 7 is a functional block diagram illustrating the structures of the C-V conversion portion 21 and a capacitor CX in the pulse-wave measurement apparatus according to the first embodiment of the present invention.

Referring to Fig. 7, the C-V conversion portion 21 includes the charging portion 51, a voltage conversion portion 52 and a voltage holding portion 53. The capacitors (the pressure detecting capacitors) CX correspond to the sensor elements 28. Herein, in Fig. 7, the multiplexer 20 is not illustrated, and only the capacitor CX selected by the multiplexer 20 is illustrated, for ease of description.

The capacitor CX changes its capacitance according to the pressure in the artery in the biologic body, at the state where the sensor array 19 in the pulse-wave measurement apparatus 100 is pressed against the biologic surface.

The charging portion 51 applies a voltage to the capacitor CX to accumulate an electric charge therein. The voltage conversion portion 52 creates a voltage resulted from conversion indicative of the capacitance of the capacitor CX, based on the electric charge accumulated in the capacitor CX, and outputs it to the voltage holding portion 53. The voltage holding portion 53 holds the voltage resulted from conversion which has been received from the voltage conversion portion 52 and outputs it to the low-pass filter 22.

Fig. 8 is a circuit diagram illustrating the structures of the C-V conversion portion 21 and a capacitor CX in the pulse-wave measurement apparatus according to the first embodiment of the present invention.

Referring to Fig. 8, the C-V conversion portion 21 is used in combination with the capacitors (the pressure detecting capacitors) CX corresponding to the sensor elements 28. The C-V conversion portion 21 includes a capacitor CC, a charge transfer capacitor CF, a capacitor CH1, a switch (first switch) SW1, a switch (fourth switch) SW4, a switch SW5, operational amplifiers G1 and G2, the charging portion 51, and the voltage setting portion 54. The charging portion 51 includes switches SW51 to SW54 and power supplies V1 and V2. The voltage setting portion 54 includes switches (a second switch and a third switch) SW2 and SW3. The switches SW1 to SW5 are MOS analog switches, for example. Further, in Fig. 8, the multiplexer 20 is not illustrated, and only the capacitor CX selected by the multiplexer 20 is illustrated, for ease of description.

In this case, the operational amplifier G1, the switch SW1, the capacitor CF and the voltage setting portion 54 correspond to the voltage conversion portion 52 illustrated in Fig. 7. Further, the switch SW5 and the capacitor CH1 correspond to the voltage holding portion 53 illustrated in Fig. 7.

The operational amplifier G1 is connected at its inverting input terminal to one end of the capacitor CX and one end of the capacitor CC and, also, is connected at its non-inverting input terminal to a ground voltage (reference voltage). The capacitor CF is connected at its one end to the inverting input terminal of the operational amplifier G1 and, also, is connected at the other end to the output of the operational amplifier G1. The switch SW1 is connected at its one end to the inverting input terminal of the operation amplifier G1 and, also, is connected at the other end to one end of the voltage setting portion 54. The voltage setting portion 54 is connected at the other end to the output of the operational amplifier G1.

In the voltage setting portion 54, the switch SW2 is connected at its one end to the other end of the switch SW1 and, also, is connected at the other end to the output of the operational amplifier G1. The switch SW3 is connected at its one end to the other end of the switch SW1 and, also, is connected at the other end to the ground voltage (the reference voltage).

The switch SW5 is connected at its one end to the output of the operational amplifier G1 and, also, is connected at the other end to one end of the capacitor CH1 and the non-inverting input terminal of the operational amplifier G2. The other end of the capacitor CH1 is connected to the ground voltage. The inverting input terminal of the operational amplifier G2 is connected to the output of the operational amplifier G2.

In the charging portion 51, the switch SW51 is connected at its one end to the positive electrode of the power supply V1 and, also, is connected at the other end to one end of the switch SW52 and the other end of the capacitor CX. The switch SW54 is connected at its one end to the negative electrode of the power supply V2 and, also, is connected at the other end to one end of the switch 53 and the other end of the capacitor CC. The other end of the switch SW52, the other end of the switch SW53, the negative electrode of the power supply V1 and the positive electrode of the power supply V2 are connected to the ground voltage. Further, the voltages outputted from the power supplies V1 and V2 have a value of VCC.

The capacitor CC is called a counter capacity and is placed for adjusting the offset of the capacitance of the capacitor CX.

The switches SW1 to SW5 are changed over between an ON state and an OFF state, based on control signals SC1 to SC5 received from the CPU 11. The switches SW51 to SW54 are changed over between an ON state and an OFF state, based on control signals received from the CPU 11, which are not illustrated.

The voltage setting portion 54 connects the other end of the switch SW1 to the output of the operational amplifier G1 or disconnects the other end of the switch SW1 from the output of the operational amplifier G1 and applies the ground voltage to the other end of the switch SW1.

### [Operations of the C-V Conversion Portion]

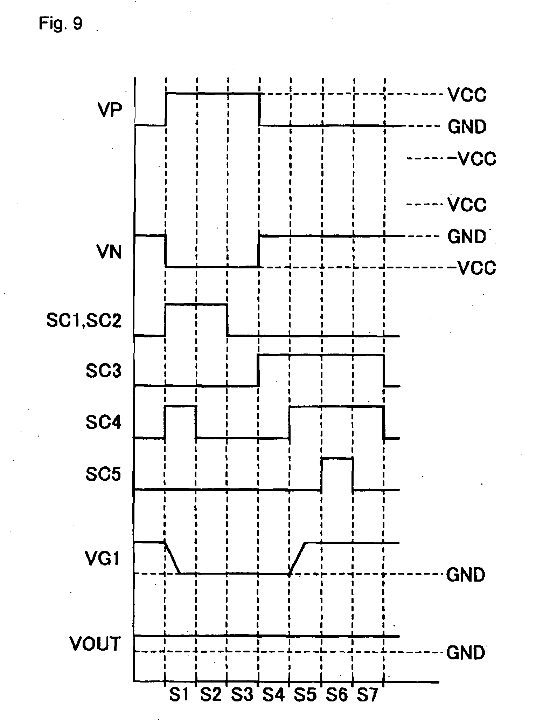

Fig. 9 is a timing chart illustrating the operations of the C-V conversion portion 21 when the pulse-wave measurement apparatus according to the first embodiment of the present invention performs pulse wave measurement. VP is a voltage applied to the other end of the capacitor CX, VN is a voltage applied to the other end of the capacitor CC, VG1 is the output voltage from the operational amplifier G1, and VOUT is the output voltage from the operational amplifier G2. When control signals SC1 to SC5 are at a high level, the switches SW1 to SW5 corresponding thereto are at an ON state, but when they are at a low level, the switches SW1 to SW5 corresponding thereto are at an OFF state.

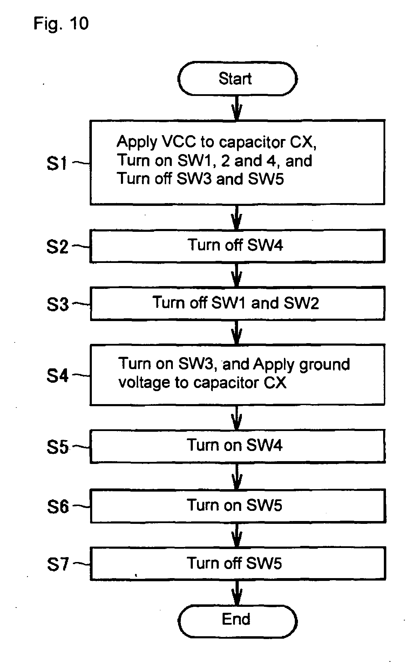

Fig. 10 is a flow chart defining the operations and procedures of the C-V conversion portion 21 when the pulse-wave measurement apparatus according to the first embodiment of the present invention performs pulse wave measurement. The CPU 11 reads programs from the ROM 22 by accessing it, decompresses the read programs in the RAM 23 and executes them to realize the processing illustrated in Fig. 10.

Referring to Figs. 9 and 10, at first, the CPU 11 turns on the switches SW1, SW2 and SW4 and, also, turns off the switches SW3 and SW5. Further, the CPU 11 turns off the switches SW52 and SW53 and, also, turns on the switches SW51 and SW54, thereby applying a charging voltage VCC to the other end of the capacitor CX and, also, applying, to the other end of the capacitor CC, a charging voltage of -VCC, namely a voltage having an absolute value equal to that of the charging voltage VCC and a direction opposite therefrom (step S1).

In this case, the voltage applied to the non-inverting input terminal of the operational amplifier G1, namely the ground voltage, is fed back from the output of the operational amplifier G1 to the inverting input terminal of the operational amplifier G1. Accordingly, an electric charge corresponding to the charging voltage VCC is accumulated in the capacitor CX and, also, an electric charge corresponding to the charging voltage -VCC is accumulated in the capacitor CC.

Next, the CPU 11 turns off the switch SW4 (step S2).
Next, the CPU 11 turns off the switches SW1 and SW2, thereby disconnecting the other end of the switch SW1 from the output of the operational amplifier G1 (step S3).

Next, the CPU 11 turns on the switch SW3, thereby applying the ground voltage (the reference voltage) to the other end of the switch SW1. Further, the CPU 11 turns on the switches SW52 and SW53 and, also, turns off the switches SW51 and SW54, thereby stopping the application of the charging voltages VCC and -VCC and, also, applying the ground voltage (the reference voltage) to the other end of the capacitor CX and the other end of the capacitor CC (step S4).

Next, the CPU 11 turns on the switch SW4. Thus, an electric charge corresponding to the difference between the amount of electric charge accumulated in the capacitor CX and the amount of electric charge accumulated in the capacitor CC is moved to the capacitor CF. Thus, the operational amplifier G1 outputs, as an output voltage G1, a voltage corresponding to the electric charge accumulated in the capacitor CF (step S5). More specifically, assuming that the capacitance of the capacitor CX is CX, the capacitance of the capacitor CC is CC, and the voltage value of the charging voltage VCC is VCC, the electric charge moved to the capacitor CF is expressed as (CX-CC)*VCC. The operational amplifier G1 converts the electric charge moved to the capacitor CF into the voltage expressed as ((CX-CC)/CF)*VCC, assuming that the capacitance of the capacitor CF is CF.

Next, the CPU 11 turns on the switch SW5. Thus, the capacitor CH1 is charged based on the output voltage from the operational amplifier G1 (step S6).

Next, the CPU 11 turns off the switch SW5. Thus, the voltage inputted to the non-inverting input terminal of the operational amplifier G2 is fixed. Then, the operational amplifier G2 outputs, as an output voltage VOUT, a voltage corresponding to the electric charge accumulated in the capacitor CH1, namely a voltage corresponding the pressure within the artery in the biologic body, to the low-pass filter 22 (step S7).

The CPU 11 repeats the processing at steps S1 to S7 to update the pressure signal outputted from the C-V conversion portion 21. Thus, the pressure waveform in the artery is determined.

On the other hand, electric-charge-to-voltage conversion type sensor devices, such as the sensor device described in Non-Patent Document 2, have had the problem of nonlinearity of the output voltage from the amplifier, namely the output characteristic of the sensor device, due to the bias-voltage dependence of the off capacity of the switch constituting the feedback circuit, such as a MOS analog switch. However, in the pulse-wave measurement apparatus according to the first embodiment of the present invention, the CPU 11 turns off the switches SW1 and SW2, thereby disconnecting the other end of the switch SW1 from the output of the operational amplifier G1. Further, the CPU 11 turns on the switch SW3, thereby applying a predetermined voltage such as the ground voltage to the other end of the switch SW1. In this case, when the switch SW3 is at an ON state, the ground voltage is fed back from the output of the operational amplifier G1 to the inverting input terminal of the operational amplifier G1, while the ground voltage is applied to one end of the switch SW1. With this structure, the bias voltage applied between the opposite ends of the switch SW1 can be maintained at a constant value during the charge transfer operation (step S5), which can prevent the nonlinearity of the output characteristic of the sensor, thereby reducing the error of pulse-wave detection.

Further, in the pulse-wave measurement apparatus according to the first embodiment of the present invention, the CPU 11 is structured to apply, to the other end of the switch SW1, the ground voltage which is the voltage applied to the non-inverting input terminal of the operational amplifier G1, which can apply the same voltage to the opposite ends of the switch SW1, thereby causing the off capacity of the switch SW1 to be substantially zero. This can further reduce the error of the output characteristic of the sensor.

Further, the pulse-wave measurement apparatus according to the first embodiment of the present invention includes the switch SW4 which is connected at its one end to one end of the capacitor CX and, also, is connected at the other end to the inverting input terminal of the operational amplifier G1. Further, the CPU 11 turns off the switch SW4, after the electric charge corresponding to the charging voltage VCC is accumulated in the capacitor CX. Then, the other end of the switch SW1 is disconnected from the output of the operational amplifier G1 and, also, the ground voltage is applied to the other end of the switch SW1. Thereafter, the CPU 11 turns on the switch SW4 to move the electric charge accumulated in the capacitor CX to the capacitor CF. By causing the bias voltage applied between the opposite ends of the switch SW1 to be a constant value and also causing the off capacity to be substantially zero, as described above, it is possible to prevent an electric charge from being moved to the off capacity of the switch SW1, when the electric charge corresponding to the difference between the amount of electric charge accumulated in the capacitor CX and the amount of electric charge accumulated in the capacitor CC is moved to the capacitor CF. This can cause an electric charge to move only to the capacitor CF, thereby improving the linearity of the sensor output.

Further, the pulse-wave measurement apparatus according to the first embodiment of the present invention employs a MOS analog switch, as the switch SW3. With this structure, in the case of forming the C-V conversion portion 21 with an integrated circuit, it is possible to constitute the switch SW3 through the same processing, thereby reducing the size of the C-V conversion portion.

Further, in the pulse-wave measurement apparatus according to the first embodiment of the present invention, the reference voltage is applied to the other end of the capacitor CX, when the application of the charging voltage is stopped. More specifically, the CPU 11 applies, to the other end of the capacitor CX, the ground voltage which is the voltage applied to the non-inverting input terminal of the operational amplifier G1, when the application of the charging voltage VCC to the other end of the capacitor CX is stopped. With this structure, it is possible to operate the operational amplifier G1 over a range within which preferable linearity is attained.

Next, another embodiment of the present invention will be described with reference to the drawings. Herein, same reference numerals are denoted for the same or corresponding components throughout the drawings, and the description thereof will not be repeated.

### <Second Embodiment>

The present embodiment relates to a pulse-wave measurement apparatus having a C-V conversion portion 21 with a changed structure.

### [The Structure of the C-V Conversion Portion]

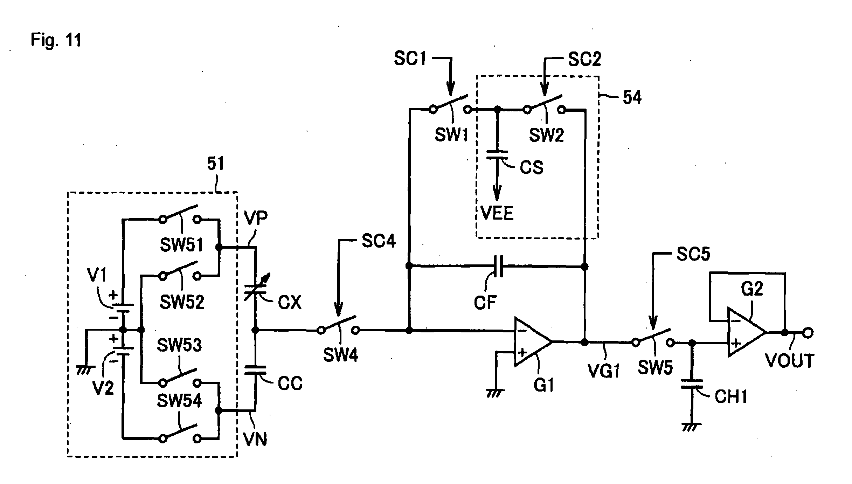

Fig. 11 is a circuit diagram illustrating the structures of the C-V conversion portion 21 and a capacitor CX in the pulse-wave measurement apparatus according to the second embodiment of the present invention.

Referring to Fig. 11, the voltage setting portion 54 according to the second embodiment of the present invention includes a capacitor (a voltage setting capacitor) CS, instead of the switch SW4.

The capacitor CS is connected at its one end to the other end of the switch SW1 and one end of the switch SW2 and, also, is connected at the other end to a predetermined voltage, such as a negative voltage of VEE.

### [Operations of the C-V Conversion Portion]

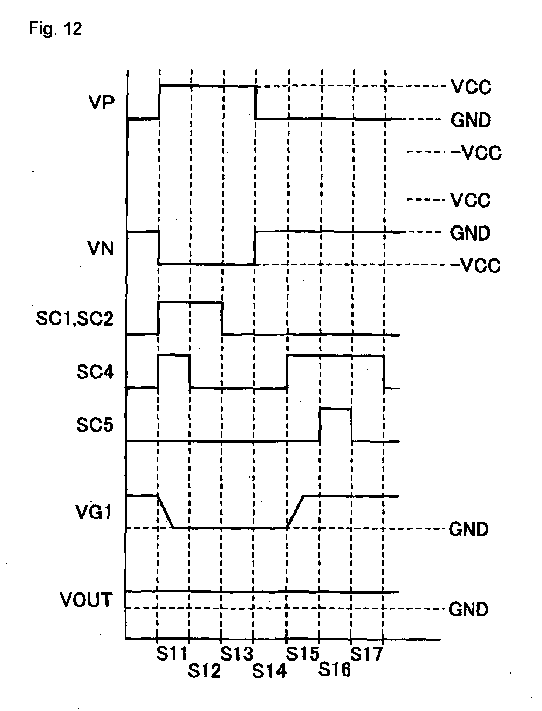

Fig. 12 is a timing chart illustrating the operations of the C-V conversion portion 21 when the pulse-wave measurement apparatus according to the second embodiment of the present invention performs pulse-wave measurement. VP is a voltage applied to the other end of the capacitor CX, VN is a voltage applied to the other end of the capacitor CC, VG1 is the output voltage from the operational amplifier G1, and the VOUT is the output voltage from the operational amplifier G2. When control signals SC1 to SC5 are at a high level, the switches SW1 to SW5 corresponding thereto are at an ON state, but when they are at a low level, the switches SW1 to SW5 corresponding thereto are at an OFF state.

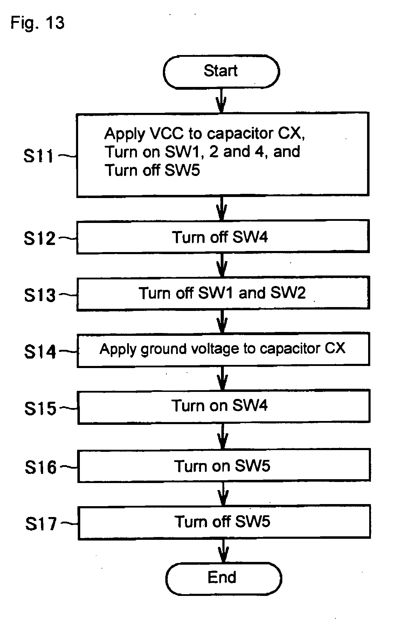

Fig. 13 is a flow chart defining the operations and procedures of the C-V conversion portion 21 when the pulse-wave measurement apparatus according to the second embodiment of the present invention performs pulse-wave measurement. The CPU 11 reads programs from the ROM 22 by accessing it, decompresses the read programs in the RAM 23 and executes them to realize the processing illustrated in the flow chart of Fig. 13.

Referring to Figs. 12 and 13, at first, the CPU 11 turns on the switches SW1, SW2 and SW4 and, also, turns off the switch SW5. Further, the CPU 11 turns off the switches SW52 and SW53 and, also, turns on the switches SW51 and SW54, thereby applying a charging voltage VCC to the other end of the capacitor CX and, also, applying, to the other end of the capacitor CC, a charging voltage of -VCC, namely a voltage having an absolute value equal to that of the charging voltage VCC and having a direction opposite therefrom (step S11).

In this case, the voltage applied to the non-inverting input terminal of the operational amplifier G1, namely the ground voltage, is fed back from the output of the operational amplifier G1 to the inverting input terminal of the operational amplifier G1. Accordingly, an electric charge corresponding to the charging voltage VCC is accumulated in the capacitor CX and, also, an electric charge corresponding to the charging voltage -VCC is accumulated in the capacitor CC.

Next, the CPU 11 turns off the switch SW4 (step S12). Further, in steps S11 and S12, the switches SW1 and SW2 are at the ON state and, therefore, an electric charge is also accumulated in the capacitor CS.

Next, the CPU 11 turns off the switches SW1 and SW2, thereby disconnecting the other end of the switch SW1 from the output of the operational amplifier G1 (step S13). In this case, the other end of the switch SW1 is maintained at the ground voltage (the reference voltage), due to the electric charge accumulated in the capacitor CS.

Next, the CPU 11 turns on the switches SW52 and SW53 and, also, turns off the switches SW51 and SW54, thereby stopping the application of the charging voltages VCC and -VCC and applying the ground voltage (the reference voltage) to the other end of the capacitor CX and the other end of the capacitor CC (step S14).

Next, the CPU 11 turns on the switch SW4. Thus, an electric charge corresponding to the difference between the amount of electric charge accumulated in the capacitor CX and the amount of electric charge accumulated in the capacitor CC is moved to the capacitor CF. Then, the operational amplifier G1 outputs, as an output voltage G1, a voltage corresponding to the electric charge accumulated in the capacitor CF (step S15). More specifically, assuming that the capacitance of the capacitor CX is CX, the capacitance of the capacitor CC is CC, and the voltage value of the charging voltage VCC is VCC, the electric charge moved to the capacitor CF is expressed as (CX-CC)*VCC. The operational amplifier G1 converts the electric charge moved to the capacitor CF into the voltage expressed as ((CX-CC)/CF)*VCC, assuming that the capacitance of the capacitor CF is CF.

Next, the CPU 11 turns on the switch SW5. Thus, the capacitor CH1 is charged based on the output voltage from the operational amplifier G1 (step S16).

Next, the CPU 11 turns off the switch SW5. Thus, the voltage inputted to the non-inverting input terminal of the operational amplifier G2 is fixed. Then, the operational amplifier G2 outputs, as an output voltage VOUT, a voltage corresponding to the electric charge accumulated in the capacitor CH1, namely a voltage corresponding the pressure within the artery in the biologic body, to the low-pass filter 22 (step S17).

The CPU 11 repeats the processing at steps S11 to S17 to update the pressure signal outputted from the C-V conversion portion 21. Thus, the pressure waveform in the artery is determined.

The other structures and operations are the same as those of the pulse-wave measurement apparatuses according to the first embodiment and will not be redundantly described in detail.

With the pulse-wave measurement apparatus according to the second embodiment of the present invention, similarly to with the pulse-wave measurement apparatuses according to the first embodiment, it is possible to prevent the nonlinearity of the output characteristic, thereby reducing the error of the pulse-wave detection.

The embodiments described herein are to be considered in all respects only as illustrative and not restrictive. The scope of the present invention is indicated by the claims rather than by the foregoing description. All changes which come within the meaning and range of equivalency of the claims are to be embraced within the scope.

## Claims

1. A pulse-wave measurement apparatus (100) for measuring pressure pulse waves in an artery by being pressed against a biologic surface, comprising:
a pressure detecting capacitor (CX) which changes its capacitance according to the pressure within the artery;
an operational amplifier (G1) which is connected at its inverting input terminal to one end of the pressure detecting capacitor (CX) and also is connected at its non-inverting input terminal to a reference voltage;
a charge transfer capacitor (CF) which is connected at its one end to the inverting input terminal of the operational amplifier (G1) and also is connected at the other end to the output of the operational amplifier (G1);
a first switch (SW1) which is connected at its one end to the inverting input terminal of the operational amplifier (G1); and **characterized by** further comprising:
a voltage setting portion (54) which is connected at its one end to the other end of the first switch (SW1) and also, is connected at the other end to the output of the operational amplifier (G1);
wherein the voltages setting portion (54) connects the other end of the first switch (SW1) to the output of the operational amplifier (G1) or disconnects the other end of the first switch (SW1) from the output of the operational amplifier (G1) and applies a predetermined voltage to the other end of the first switch (SW1).

2. The pulse-wave measurement apparatus according to Claim 1,
wherein the voltage setting portion (54) includes a second switch (SW2) which is connected at its one end to the other end of the first switch (SW1) and also is connected at the other end to the output of the operational amplifier (G1), and a third switch (SW3) which is connected at its one end to the other end of the first switch (SW1) and also is connected at the other end to the predetermined voltage.

3. The pulse-wave measurement apparatus according to Claim 1,
wherein the voltage setting portion (54) includes a second switch (SW2) which is connected at its one end to the other end of the first switch (SW1) and also is connected at the other end to the output of the operational amplifier (G1), and a voltage setting capacitor (CS) which is connected at its one end to the other end of the first switch (SW1) and also is connected at the other end to the predetermined voltage.

4. The pulse-wave measurement apparatus (100) according to Claim 1, further comprising a charging portion (51) for applying a charging voltage to the other end of the pressure detecting capacitor (CX) and a control portion (11),
wherein the control portion (11) applies the charging voltage to the other end of the pressure detecting capacitor (CX), turns on the first switch (SW1) and connects the other end of the first switch (SW1) to the output of the operational amplifier (G1), then disconnects the other end of the first switch (SW1) from the output of the operational amplifier (G1), then applies the predetermined voltage to the other end of the first switch (SW1) and also stops the application of the charging voltage, by controlling the charging portion (51), the first switch (SW1) and the voltage setting portion (54).

5. The pulse-wave measurement apparatus (100) according to Claim 1, further comprising a fourth switch which is connected at its one end to one end of the pressure detecting capacitor (CX) and also connected at the other end to the inverting input terminal of the operational amplifier (G1).

6. The pulse-wave measurement apparatus (100) according to Claim 5, further comprising a charging portion (51) for applying a charging voltage to the other end of the pressure detecting capacitor (CX) and a control portion (11),
wherein the control portion (11) applies the charging voltage to the other end of the pressure detecting capacitor (CX), turns on the first switch (SW1), turns on the fourth switch (SW4), connects the other end of the first switch (SW1) to the output of the operational amplifier (G1), then turns off the fourth switch (SW4), then disconnects the other end of the first switch (SW1) from the output of the operational amplifier (G1), then applies the predetermined voltage to the other end of the first switch (SW1) and also stops the application of the charging voltage, and then turns on the fourth switch (SW4), by controlling the charging portion (51), the first switch (SW1), the fourth switch (SW4) and the voltage setting portion (54).

7. The pulse-wave measurement apparatus according to Claim 1, wherein the predetermined voltage is the reference voltage.

## Patentansprüche

1. Eine Pulswellenmessvorrichtung (100) zur Messung von Druckpulswellen in einer Arterie, wobei die Pulswellenmessvorrichtung zur Messung auf eine biologische Oberfläche gedrückt wird, aufweisend:
einen druckdetektierenden Kondensator (CX), der seine Kapazität gemäß dem Druck innerhalb der Arterie ändert;
einen Operationsverstärker (G1), der mit seinem invertierenden Eingang mit einem Ende des druckdetektierenden Kondensators (CX) verbunden ist und der außerdem mit seinem nicht invertierenden Eingang mit einer Referenzspannung verbunden ist;
einen Ladungsübertragungskondensator (CF), der mit seinem einen Ende mit dem invertierenden Eingang des Operationsverstärkers (G1) verbunden ist und der außerdem mit dem anderen Ende mit dem Ausgang des Operationsverstärkers (G1) verbunden ist;
einen ersten Schalter (SW1), der mit seinem einen Ende mit dem invertierenden Eingang des Operationsverstärkers (G1) verbunden ist; und wobei die Vorrichtung **dadurch gekennzeichnet ist, dass** sie weiterhin aufweist:
einen Spannungseinstellungsabschnitt (54), der mit seinem einen Ende mit dem anderen Ende des ersten Schalters (SW1) verbunden ist und der außerdem mit seinem anderen Ende mit dem Ausgang des Operationsverstärkers (G1) verbunden ist;
wobei der Spannungseinstellungsabschnitt (54) das andere Ende des ersten Schalters (SW1) mit dem Ausgang des Operationsverstärkers (G1) verbindet oder das andere Ende des ersten Schalters (SW1) von dem Ausgang des Operationsverstärkers (G1) trennt und eine vorbestimmte Spannung an das andere Ende des ersten Schalters (SW1) anlegt.

2. Die Pulswellenmessvorrichtung gemäß Anspruch 1, wobei der Spannungseinstellungsabschnitt (54) einen zweiten Schalter (SW2), der mit seinem einen Ende mit dem anderen Ende des ersten Schalters (SW1) verbunden ist und der außerdem mit seinem anderen Ende mit dem Ausgang des Operationsverstärkers (G1) verbunden ist, und einen dritten Schalter (SW3), der mit seinem einen Ende mit dem anderen Ende des ersten Schalters (SW1) verbunden ist und der außerdem mit dem anderen Ende mit der vorbestimmten Spannung verbunden ist, aufweist.

3. Die Pulswellenmessvorrichtung gemäß Anspruch 1, wobei der Spannungseinstellungsabschnitt (54) einen zweiten Schalter (SW2), der mit seinem einen Ende mit dem anderen Ende des ersten Schalters (SW11) verbunden ist und der außerdem mit dem anderen Ende mit dem Ausgang des Operationsverstärkers (G1) verbunden ist, und einen Spannungseinstellungskondensator (CS), der mit seinem einen Ende mit dem anderen Ende des ersten Schalters (SW1) verbunden ist und der außerdem mit dem anderen Ende mit der vorbestimmten Spannung verbunden ist, aufweist.

4. Die Pulswellenmessvorrichtung (100) gemäß Anspruch 1, weiterhin aufweisend einen Ladeabschnitt (51) zum Anlegen einer Ladespannung an das andere Ende des druckdetektierenden Kondensators (CX) und einen Steuerungsabschnitt (11), wobei
der Steuerungsabschnitt (11) durch Steuerung des Ladeabschnitts (51), des ersten Schalters (SW11) und des Spannungseinstellungsabschnitts (54) die Ladespannung an das andere Ende des druckdetektierenden Kondensators (CX) anlegt, den ersten Schalter (SW11) einschaltet und das andere Ende des ersten Schalters (SW1) mit dem Ausgang des Operationsverstärkers (G1) verbindet, anschließend das andere Ende des ersten Schalters (SW1) von dem Ausgang des Operationsverstärkers (G1) trennt, anschließend die vorbestimmte Spannung an das andere Ende des ersten Schalters (SW1) anlegt und ebenfalls das Anlegen der Ladespannung beendet.

5. Die Pulswellenmessvorrichtung (100) gemäß Anspruch 1, weiterhin aufweisend einen vierten Schalter, der mit seinem einen Ende mit dem einen Ende des druckdetektierenden Kondensators (CX) verbunden ist und der außerdem mit dem anderen Ende mit dem invertierenden Eingang des Operationsverstärkers (G1) verbunden ist.

6. Die Pulswellenmessvorrichtung (100) gemäß Anspruch 5, weiterhin aufweisend einen Ladeabschnitt (51) zum Anlegen einer Ladespannung an das andere Ende des druckdetektierenden Kondensators (CX) und einen Steuerungsabschnitt (11), wobei
der Steuerungsabschnitt (11) durch Steuerung des Ladeabschnitts (51), des ersten Schalters (SW11), des vierten Schalters (SW4) und des Spannungseinstellungsabschnitts (54) die Ladespannung an das andere Ende des druckdetektierenden Kondensators (CX) anliegt, den ersten Schalter (SW1) einschaltet, den vierten Schalter (SW14) einschaltet, das andere Ende des ersten Schalters (SW1) mit dem Ausgang des Operationsverstärkers (G1) verbindet, anschließend den vierten Schalter (SW14) ausschaltet, anschließend das andere Ende des ersten Schalters (SW1) von dem Ausgang des Operationsverstärkers (G1) trennt, anschließend die vorbestimmte Spannung an das andere Ende des ersten Schalters (SW1) anlegt und ebenfalls das Anlegen der Ladespannung beendet und anschließend den vierten Schalter (SW4) einschaltet.

7. Die Pulswellenmessvorrichtung gemäß Anspruch 1, wobei die vorbestimmte Spannung die Referenzspannung ist.

## Revendications

1. Appareil de mesure d'ondes de pression (100) pour mesurer des ondes de pression dans une artère en étant appuyé contre une surface biologique, comprenant :
• un condensateur de détection de pression (CX) qui change sa capacité électrique selon la pression de l'artère ;
• un amplificateur opérationnel (G1) qui est raccordé à son terminal d'entrée inverseuse à une extrémité du condensateur de détection de pression (CX) et est également raccordé à son terminal d'entrée non inverseuse à une tension de référence ;
• un condensateur de transfert de charge (CF) qui est raccordé à l'une de ses extrémités au terminal d'entrée inverseuse de l'amplificateur opérationnel (G1) et est également raccordé à l'autre extrémité à la sortie de l'amplificateur opérationnel (G1) ;
• un premier commutateur (SW1) qui est raccordé a l'une de ses extrémités au terminal d'entrée: inverseuse de l'amplificateur opérationnel (G1) ; et **caractérisé en ce qu'**il comprend en outre :
• une partie d'établissement de tension (54) qui est raccordée à l'une des ses extrémités à l'autre extrémité du premier commutateur (SW1) et est également raccordée à l'autre extrémité à la sortie de l'amplificateur opérationnel (G1) ;
• la partie d'établissement de tension (54) raccordant l'autre extrémité du premier commutateur (SW1) à la sortie de 11 amplificateur opérationnel (G1) ou déconnectant l'autre extrémité du premier commutateur (SM1) de la sortie de l'amplificateur opérationnel (G1) et appliquant une tension prédéterminée à l'autre extrémité du premier commutateur (SW1).

2. Appareil de mesure d'ondes de pression selon la revendication 1, dans lequel la partie d'établissement de tension (54) comprend un deuxième commutateur (SW2) qui est raccordé à l'une de ses extrémités à l'autre extrémité du premier commutateur (SW1) et est également raccordé à l'autre extrémité à la sortie de l'amplificateur opérationnel (G1) et un troisième commutateur (SW3:) qui est raccordé à l'une de ses extrémités à l'autre extrémité du premier commutateur (SW1) et est également raccordé à l'autre extrémité à une tension prédéterminée,

3. Appareil de mesure d'ondes de pression selon la revendication 1, dans lequel la partie d'établissement de tension (54) comprend un deuxième commutateur (SW2) qui est raccordé à l'une de ses extrémité à l'autre extrémité, du premier commutateur (SW1) et également est raccordé à l'autre extrémité à la sortie de l'amplificateur opérationnel (G1) et un condensateur d'établissement de tension (c) qui est raccordé à l'une de ses extrémités à l'autre extrémité du premier commutateur (SW1) et est également raccordé à l'autre extrémité à la tension prédéterminée.

4. Appareil de mesure d'ondes de pression (100) selon la revendication 1, comprenant en outre une partie de charge (51) pour appliquer une tension de charge à l'autre extrémité du condensateur de détection de pression (CX) et une partie de commande (11), dans lequel la partie de commande (11) applique la tension de charge à l'autre extrémité du condensateur de détection de pression (CX), active le premier commutateur (SW1) et raccorde l'autre extrémité du premier commutateur (SW1) à la sortie de l'amplificateur opérationnel (G1) puis déconnecte, l'autre extrémité du premier commutateur (SW1) de la sortie de l'amplificateur opérationnel (G1), puis applique une tension prédéterminée à l'autre extrémité du premier commutateur (SW1) et arrête également l'application de la tension de charge en contrôlant la partie de charge (51) , le premier commutateur (SW1) et la partie d'établissement de tension (54).

5. Appareil de mesure d'ondes de pression (160) selon la revendication 1, comprenant en outre un quatrième commutateur qui est raccorde à l'une de ses extrémités à une extrémité du condensateur de détection de pression (CX) et est également raccordé à l'autre extrémité au terminal d'entrée seule de l'amplificateur opérationnel (G1).

6. Appareil de mesure d'ondes de pression (100) selon la revendication 5, comprenant en outre une partie de charge (51) pour appliquer une tension de charge à l'autre extrémité du condensateur de détection de pression (CX) et une partie de commande (11), dans lequel la partie de commande (11) applique, la tension de charge à l'autre extrémité du condensateur de détection de pression (CX), active le premier commutateur (SW1), active le quatrième commutateur (SW4), raccorde l'autre extrémité du premier commutateur (SW1) à la sortie de l'amplificateur opérationnel (G1) puis désactive le quatrième commutateur (SW4), puis déconnecte l'autre extrémité du premier commutateur (SW1) de la sortie de l'amplificateur opérationnel (G1), puis applique une tension prédéterminée à l' autre extrémité du premier commutateur (SW1) et arrête également l'application de la tension de charge puis active le quatrième commutateur (SW4) en contrôlant la partie de charge (51), le premier commutateur (SW1), le quatrième commutateur (SW4) et la partie d'établissement de tension (54).

7. Appareil de mesure d'ondes de pression selon la revendication 1, dans lequel la tension prédéterminée est la tension de référence. ²
